# EUROPÄISCHE PATENTSCHRIFT

(11) **EP 1 335 750 B1**
(45) Veröffentlichungstag und Bekanntmachung des Hinweises auf die Patenterteilung: **06.07.2005**
(21) Anmeldenummer: 01997259.5
(22) Anmeldetag: 22.11.2001
(51) Int. Cl.: A61K 49/00

(54) **ALLERGIE-TESTKAMMER**
ALLERGY TEST CHAMBER
CHAMBRE DE TEST D'ALLERGIE

(30) Priorität: 23.11.2000 AT 19742000
(43) Veröffentlichungstag der Anmeldung: 20.08.2003
(73) Patentinhaber: Horak, Friedrich, 3052 Innermanzing (AT)
(72) Erfinder: Horak, Friedrich, 3052 Innermanzing (AT)
(74) Vertreter: Ellmeyer, Wolfgang
(86) Internationale Anmeldenummer: PCT/AT2001/000370
(87) Internationale Veröffentlichungsnummer: WO 2002/041784

(56) Entgegenhaltungen:
- RONBORG S M ET AL: "Exposure chamber for allergen challenge. The development and validation of a new concept." ALLERGY. DENMARK FEB 1996, Bd. 51, Nr. 2, Februar 1996 (1996-02), Seiten 82-88, XP008007828 ISSN: 0105-4538
- HORAK F ET AL: "Efficacy and tolerability of astemizole-D and Loratadine-D during prolonged, controlled allergen challenge in the Vienna Challenge Chamber." ARZNEIMITTEL-FORSCHUNG. GERMANY NOV 1996, Bd. 46, Nr. 11, November 1996 (1996-11), Seiten 1077-1081, XP002212836 ISSN: 0004-4172
- HTUT TIN ET AL: "A pilot study on the effect of one room mechanical ventilation with heat recovery (MVHR) units on house dust mite populations and Der p 1 levels in laboratory simulated bedrooms and on ambient conditions in an occupied bedroom in Cambridge, UK." INTERNATIONAL JOURNAL OF ENVIRONMENTAL HEALTH RESEARCH, Bd. 6, Nr. 4, 1996, Seiten 301-313, XP008007826 ISSN: 0960-3123

## Beschreibung

Die Erfindung betrifft eine Allergie-Testkammer, mit zumindest einem Einlaß zur Zufuhr von allergenfreier Luft, insbesondere Frischluft, in das Kammerinnere.

### Hintergrund der Erfindung

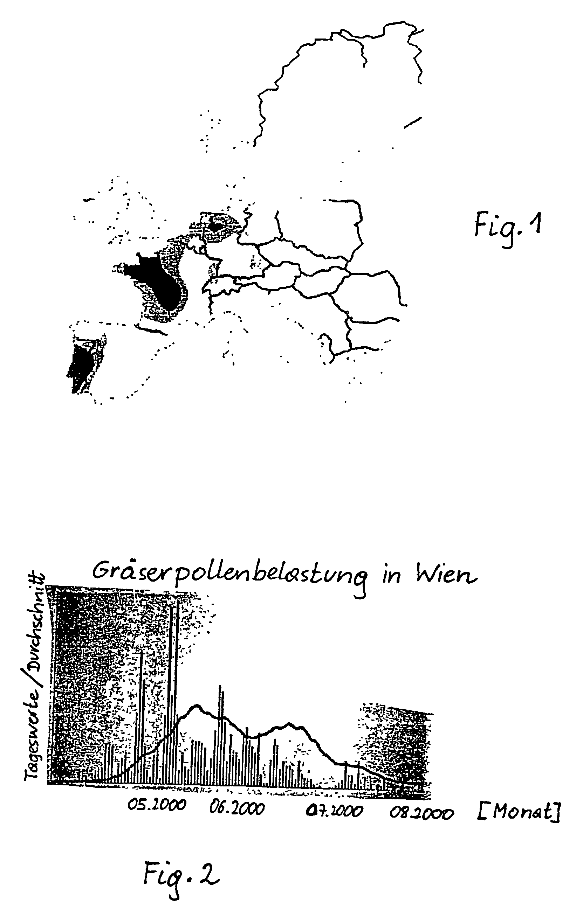

Die Wirksamkeit einer Antiallergenbehandlung kann nur dann objektiv beurteilt werden, wenn allergische Patienten unter der natürlichen Belastung eines spezifischen Allergens, wie Graspollen, beobachtet werden. Die mögliche Nutzung der Daten, die im Rahmen einer solchen unter Umweltbedingungen durchgeführten klinischen Studie erhalten wurden, ist aufgrund der extremen Schwankung der Allergenkonzentration in der Luft stark eingeschränkt. Sie ändert sich nicht nur von Region zu Region, wie in Fig. 1 anhand der Graspollenkonzentration in Europa vom 10. Juni 2000 dargestellt, wobei die dunklen Flächen eine hohe Konzentration und die hellen Flächen eine niedrige Pollenkonzentration repräsentieren, von Jahr zu Jahr und innerhalb einer Jahreszeit (siehe Fig. 2, die saisonale Schwankungen und den Durchschnitt über 20 Jahre in der Graspollenzahl zeigt), sondern auch im Verlauf eines Tages und ganz wesentlich mit dem Abstand vom Boden. Darüber hinaus unterscheiden sich die Reaktionen von Patienten auf Allergene in der Luft aufgrund einer Priming-Wirkung und anderer Phänomene von eher sekundärer Natur deutlich im Verlauf einer Jahreszeit. Diese Probleme treten in subjektiv tolerierbaren Symptomen, wie Konjunktivitis, deutlicher zu Tage, weniger bei Rhinitis und selten in schweren Anfällen, wie Asthma.

Ein weiteres Problem ist die konsistente Aufzeichnung über einen Zeitraum einiger Tage, wie sie beinahe nur durch eine subjektive Bewertung der Symptome erfolgen kann. Die Zuverlässigkeit der Dokumentation in von Patienten geführten Tagebüchern nimmt mit jedem Untersuchungstag ab. Um statistisches Material zu erhalten, das in Untersuchungen für die Dosis-Findung, Wirksamkeit oder Wirkungsdauer verwendet werden kann, sind eine große Zahl von Patienten und lange Beobachtungszeiträume notwendig. Jedoch können eine niedrige Pollenzahl und viele andere Umweitfaktoren bei Untersuchungen im Feld zu unerwartet insignifikanten Ergebnissen führen.

Daher ist es notwendig, eine Lösung für klinische Versuche mit Patienten mit Pollen- und Hausstaubmilbenallergien zu finden, die Feldversuchen nahe kommen, nicht aber deren Nachteile aufweisen.

### Stand der Technik

Zur Zeit sind Tests in Allergen-Testkammern unwidersprochen als "Golden Standard" bekannt, mit dem sich die oben angeführten Probleme überwinden lassen (Cartier A., et al.: "Guidelines for bronchoprovocation on the investigation of occupational asthma", Report of the Subcommittee on Provocation for Occupational Asthma. J Allergy Clin Immunol 1989, 84:823-829); Melillo G.: "European Academy of Allergy and Clinical Immunology - Provocation tests with allergens", Allergy 1997, Suppl. 35,52:1-35). Diese Kammern ermöglichen Test mit der Inhalation von Allergenen unter kontrollierten Bedingungen, die natürlicher Belastung nahe kommen und weiters präzise und umfassende Ergebnisse für jeden Patienten garantieren. Da eine Allergen-Testkammer genaue Untersuchungen ermöglicht, während sie eine große Anzahl nummerischer Daten hervorbringt, ermöglicht sie es dem Forscher, statistisch signifikante Ergebnisse mit einer weit geringeren Anzahl von Patienten zu erhalten, als in einem Feldversuch notwendig wäre.

Die bisher weitest entwickelte Allergie-Testkammer besteht aus einem gegenüber Außenluft verschließbaren Raum, in dem eine Anzahl von Ventilatoren aufgestellt ist, die die Raumluft waagrecht im Raum zirkulieren lassen. Allergene, wie z.B. Graspollen oder Milbenkot, werden in die Raumluft eingebracht, indem eine oder mehrere Schalen mit einer vorbestimmten Menge an Allergenen vor jeweiligen Ventilatoren aufgestellt werden und durch den Luftzug des Ventilators verwirbelt werden. Der solcherart erreichten relativ ungleichmäßigen Verteilung der Allergene über den Raum versucht man in Tests dadurch zu begegnen, daß die Versuchspersonen angewiesen sind, in regelmäßigen zeitlichen Abständen ihre Plätze im Raum zu wechseln.

Ronborg et Al. (Allergy, 1996, Bd. 51, Nr. 2, 82-88) und Horak et Al. (Wien. Klin. Wochenschr., 1987, Nr. 14, 509-510) beschreiben beide eine Allergie-Testkammer wobei der Frischluft primär allergenbeladene Zuluft ausserhalb der Kammer beigemischt wird.

Es wäre wünschenswert, eine Allergie-Testkammer zur Verfügung zu haben, die eine wesentlich verbesserte Gleichförmigkeit der Allergenbeladung der Luft im Raum garantiert und so die Objektivierbarkeit und Wiederholbarkeit von Tests mit Versuchspersonen weiter verbessert.

### Erfindungsgemäße Lösung

Diese Aufgabe wird erfindungsgemäß durch Fortbildung der eingangs genannten Allergie-Testkammer auf solche Weise gelöst, daß der zumindest eine Einlaß für allergenfreie Luft mit einer Luftstrom-Verteilungseinrichtung versehen ist und daß die Allergie-Testkammer weiters zumindest einen Einlaß für Allergen-Testpartikel, wie z.B. Pflanzen pollen oder Milbenkot, und zumindest einen Auslaß zur Absaugung von mit Allergen-Testpartikeln beladener Luft aus dem Kammerinneren aufweist, wobei der Auslaß von den Einlässen für allergenfreie Luft und Allergen-Testpartikel beabstandet ist. Es hat sich überraschenderweise gezeigt, daß die Abkehr vom bekannten und oben beschriebenen Prinzip der Zirkulation von mit Allergenen beladenen Luft in der Testkammer zugunsten eines Durchzugs-Prinzips, bei der die Luft in die Kammer eingebracht, mit einer definierten Menge an Allergenen vermischt und die mit Allergenen beladene Luft an anderer Stelle der Kammer wieder abgesaugt wird, zu einer gegenüber dem Stand der Technik wesentlich verbesserten Gleichförmigkeit der Verteilung der Allergen-Partikel in der Testkammer führt, wie weiter unten anhand eines Diagramms näher erläutert.

Eine-baulich leicht zu verwirklichende Ausführungsform der Erfindung ist dadurch gekennzeichnet, daß der zumindest eine Einlaß für allergenfreie Luft ein Luftzufuhrrohr mit einer Vielzahl von Durchgangsöffnungen in der Rohrwand umfaßt.

Um eine ausgezeichnet gleichmäßige Verteilung und gegebenenfalls Verwirbelung bzw. Erzeugung einer turbulenten Strömung der Luft in der Allergie-Testkammer zu erreichen, kann vorgesehen sein, die Luftstrom-Verteilungseinrichtung mit in verschiedene Richtungen gerichteten Luftdüsen, vorzugsweise Kugeldüsen, zu versehen. Alternativ dazu kann die Luftstrom-Verteilungseinrichtung Ablenkbleche oder Luftverteiler-Kämme umfassen.

Die Einlässe zur Zufuhr von Allergen-Testpartikeln und von allergenfreier Luft sind bevorzugt voneinander beabstandet, können allerdings in einer anderen Ausführungsform auch ineinander integriert sein, wenn für geeignete Strömungspfade der Luft um die Einlässe zur Zufuhr der Testpartikel gesorgt ist.

Bei einer bevorzugten Ausführungsform der erfindungsgemäßen Allergie-Testkammer sind die Einlässe zur Zufuhr von Allergen-Testpartikeln und von allergenfreier Luft in der Decke der Kammer und/oder in zumindest einer Seitenwand der Kammer in Deckennähe angeordnet und ist der zumindest eine Auslaß zur Absaugung von mit Allergen-Testpartikeln beladener Luft im Boden der Kammer und/oder in zumindest einer Seitenwand der Kammer in Bodennähe angeordnet.

Um für beste Luftgüte in der Kammer zu sorgen ist es zweckmäßig, in den Strömungsweg der allergenfreien Luft in die Kammer zumindest ein Mehrschicht-Filtersystem zur Reduzierung von Schadstoffen zwischenzuschalten.

Allergen-Testpartikel neigen bei erhöhter Luftfeuchtigkeit zur Verklumpung. Damit solche Verklumpungen nicht die klinischen Tests beeinflussen, ist es zweckmäßig eine Vorrichtung zur Deaglomerierung der in die Kammer einzubringenden Allergenpartikel vorzusehen.

Um das weitere Ziel eines halb- oder vollautomatischen Betriebs der Allergie-Testkammer zu erreichen, ist gemäß der Erfindung bevorzugt eine Vorrichtung zur automatischen Dosierung von Allergen-Testpartikeln und ihrer kontinuierlichen oder intermittierenden Beförderung in die Kammer vorgesehen, wobei zur Beförderung der Partikel vorzugsweise Druck- oder Unterdrucksysteme dienen. Die Vorrichtung zur automatischen Dosierung kann Mikro-Schneckenförderer, Mikro-Zahnradförderer, Mikro-Archimedes-Schraubenförderer, Mikro-Rotations-Siebscheiben, Zentrifugalselektion, elektromagnetische oder elektrostatische Dosierfelder umfassen. Ein halbautomatischer Betrieb kann unter Einsatz von Mikro-Dosierlöffeln realisiert werden.

Um eine besonders gleichmäßige Einbringung der Allergen-Testpartikel in die Allergie-Testkammer zu gewährleisten, ist an den zumindest einen Einlaß für Allergen-Testpartikel ein Partikel-Verteiler anschließbar, wobei der Partikel-Verteiler vorzugsweise so anbringbar ist, daß die Allergen-Testpartikel an den Punkten mit Strömungsinterferenz Null der allergenbeladenen Luft eingebracht werden. Eine bevorzugte Ausführungsform eines Partikel-Verteilers weist einen Körper mit einer Axialbohrung und sich von der Axialbohrung zur Außenperipherie des Verteilers erstreckende Radialbohrungen auf. Alternativ dazu kann der Partikelverteiler einen Körper mit gerichteten kammartigen Austrittsöffnungen aufweisen. Weiters ist es zweckmäßig, wenn der Partikel-Verteiler eine Prallplatte nahe dem Einlaß für Allergen-Testpartikel aufweist, wobei die Allergen-Testpartikel mittels Druckluft durch den Einlaß befördert werden.

Um für die klinischen Tests vorgegebene und reproduzierbare Luftbedingungen zu schaffen, ist in einer Ausführungsform der Erfindung ein Klimamodul zur Steuerung von Klimaparametern wie Temperatur, Feuchtigkeit, Luftumsatz etc. der in das Kammerinnere zugeführten allergenfreien Luft vorgesehen, wobei das Klimamodul vorzugsweise über einen Echtzeit-Regelkreis zur Steuerung der Parameter verfügt.

Damit der Austritt von Allergen-Testpartikeln durch Öffnungen in der Allergie-Testkammer, wie z.B. Türen oder Durchreichöffnungen verhindert wird, ist der barometrische Druck in der Kammer gegenüber dem barometrischen Druck außerhalb der Kammer reduziert. Die Druckdifferenz beträgt vorzugsweise 10 - 200 Pa.

Die erfindungsgemäße Allergie-Testkammer weist bevorzugt zumindest ein Meßgerät zur ldentifizierung repräsentativer Parameter der eingebrachten Allergen-Testpartikeln auf. Dabei können Meßgeräte zur Messung der Partikel-Konzentration der Luft im Kammerinneren vorgesehen sein. Zur Messung der individuellen Allergen-Belastung sollte für jede Versuchsperson in der Kammer zumindest ein Meßgerät bereitgestellt werden. Bevorzugt ist weiters zumindest ein Partikel-Sammelgerät zur Kumulierung der über eine Zeiteinheit eingebrachten absoluten Partikel-Menge zwecks anschließender Analyse essentieller Allergenparameter vorgesehen.

Die Erfindung wird nun anhand eines nicht einschränkenden Ausführungsbeispiels näher erläutert. In den Zeichnungen zeigen Fig. 1 die Graspollenkonzentration in Europa vom 10. Juni 2000, wobei dunklere Flächen höhere Konzentrationen darstellen, Fig. 2 die saisonale Schwankung und den Durchschnitt über 20 Jahre in der Graspollenzahl, Fig. 3 einen Grundriß der Testkammer und des sie umgebenden Arbeitsraums, Fig. 4 einen Aufriß der Testkammer, Fig. 5 die Verteilung der Pollen-Beladung in einer Höhe von 1,5 m in der erfindungsgemäßen Testkammer, Fig. 6 die Schwankung der Pollen-Beladung während einer 6 h dauernden Testsitzung in der Testkammer, Fig. 7 eine perspektivische Darstellung einer erfindungsgemäßen Belüftungsleiste mit Kugeldüsen, Figuren 8a und 8b einen erfindungsgemäßen Partikel-Verteiler in Längs- und Querschnitt, Fig. 9 ein Diagramm der Schwankung der Graspollenzahl während 4 h im Feld und in der erfindungsgemäßen Testkammer, und Fig. 10 ein Diagramm von Aufzeichnungen des subjektiven Augen-Juckens von Testpersonen im Feld und der erfindungsgemäßen Testkammer.

### Aufbau der Kammer

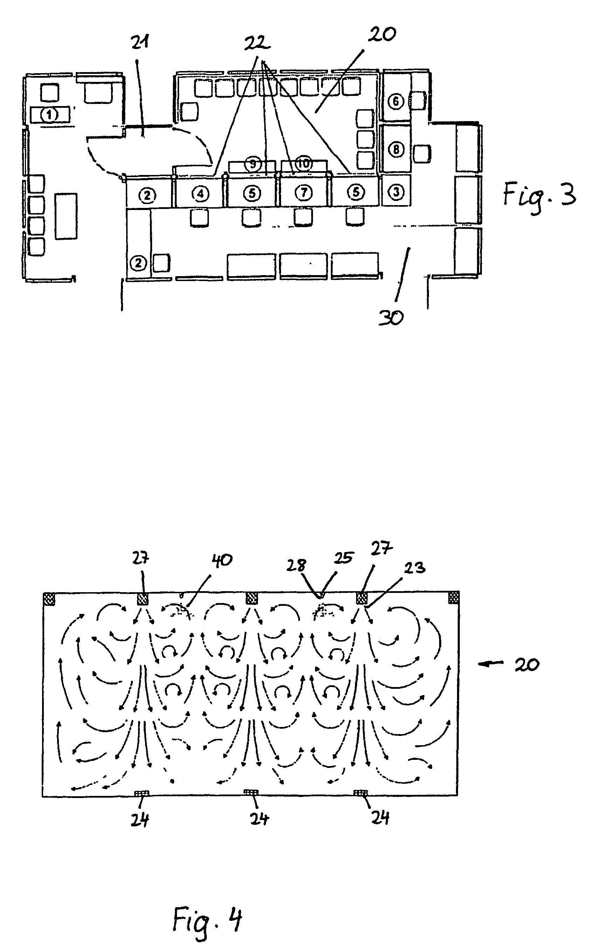

Die erfindungsgemäße Allergen-Testkammer 20 (im folgenden auch als VCC für "Vienna Challenge Chamber" abgekürzt) ist ein abgeschlossenes System, das mit innenraum-Luft befüllt ist. Mit ihrer Länge von 5,25 m und Breite von 2,60 m umfasst die VCC eine Fläche von 13,65 m², und bei einer durchschnittlichen Höhe von 2,70 m hat sie ein Volumen von etwa 37,20 m³. Die Allergen-Testkammer 20 ist in Fig. 3 gemeinsam mit dem anschließenden Arbeitsbereich 30 im Grundriß dargestellt und in Fig. 4 im Aufriß zu sehen. In die VCC 20 gelangt man durch eine 1,30 m² große Luftschleuse 21 mit Pollen-Abstreifmatten bzw. einem "Sumpf" am Boden. In der Kammer 20 finden bis zu vierzehn Patienten Platz, die gleichzeitig für mehrere Stunden (üblicherweise 2 bis 8 h, unter Berücksichtigung des Versuchsziels) unter kontrollierten und reproduzierbaren Bedingungen getestet werden können. Vier Fenster 22 zwischen der Kammer 20 und dem umgebenden Arbeitsbereich 30 ermöglichen die konstante Beobachtung und Untersuchung bzw. Befragung der getesteten Patienten. Die Zahlen (1) bis (10) bezeichnen Untersuchungs- bzw. Testplätze, von denen sich die Plätze (1) bis (8) im Arbeitsbereich 30 und die Plätze (9) und (10) in der Testkammer 20 befinden. Glatte Aluminium-Oberflächen, Antistatik-Maßnahmen und regelmäßige gründliche Reinigung zwischen den Testsitzungen verringern die Allergen-Anhaftung, die zu unregelmäßigen Allergenkonzentrationen führen könnte.

### Belüftung

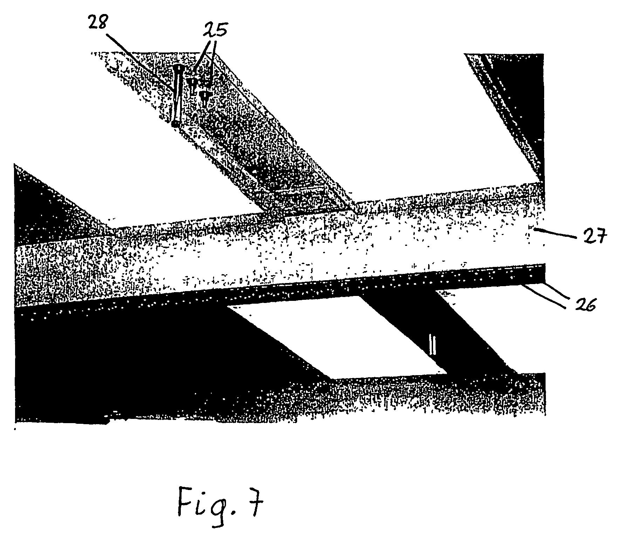

Ein automatisches Luftzufuhrsystem in die Kammer garantiert permanente und gleichmäßige Klimatisierung. Die Temperatur, üblicherweise in einem Bereich von 24 bis 26 °C, wird online (alle 5 s) an vier verschiedenen Stellen der Kammer 20 in einer Höhe von 2,2 m aufgezeichnet, ebenso wie die relative Luftfeuchtigkeit, die üblicherweise in einem Bereich von 4 bis 45 % gehalten wird. Eine stabile Temperatur wird ca. 15 min nach dem Beginn der Testsitzung erreicht, wobei es nur eine Schwankung im Bereich von 0,5 °C und 1,5 % Feuchtigkeit gibt. Weiters wird die CO₂-Konzentration online (alle 5 s) an einem einzigen Punkt 0,5 m über dem Boden aufgezeichnet. Die CO₂-Konzentration wird während der gesamten Testsitzung unabhängig von der Anzahl getesteter Patienten und der Testdauer im Bereich von 0,1 % gehalten. Allergenfreie Luft wird durch 384 Einlässe 23 in der Decke in die Kammer 20 geblasen, was zu einer konstanten und leicht turbulenten Luftströmung führt, wie in Fig. 4 dargestellt. Die 384, in Fig. 4 symbolisch dargestellten Einlässe 23 werden durch Kugeldüsen 26 gebildet (siehe Fig. 7), die in der Wand eines Luftzufuhrrohr 27 montiert sind. Die Kugeldüsen sind in verschiedene Richtungen gerichtet, die für die turbulente Strömung sorgen. Die turbulente Luftströmung wiederum führt zu homogener Allergen-Testpartikel-Verteilung in der Kammer. Verbrauchte Luft wird durch sechs Auslässe 24 nahe dem Boden abgesaugt. Die zugeführte Frischluftmenge wird in Bezug auf die Anzahl an Patienten in der Testkammer kalibriert. Um zu garantieren, dass keine Luft aus der Testkammer den Arbeitsbereich oder andere Teile des Gebäudes kontaminiert, wird der Druck innerhalb der Kammer etwa 60 Pa niedriger als außerhalb gehalten.

### Allergen-Verteilung in der Kammer

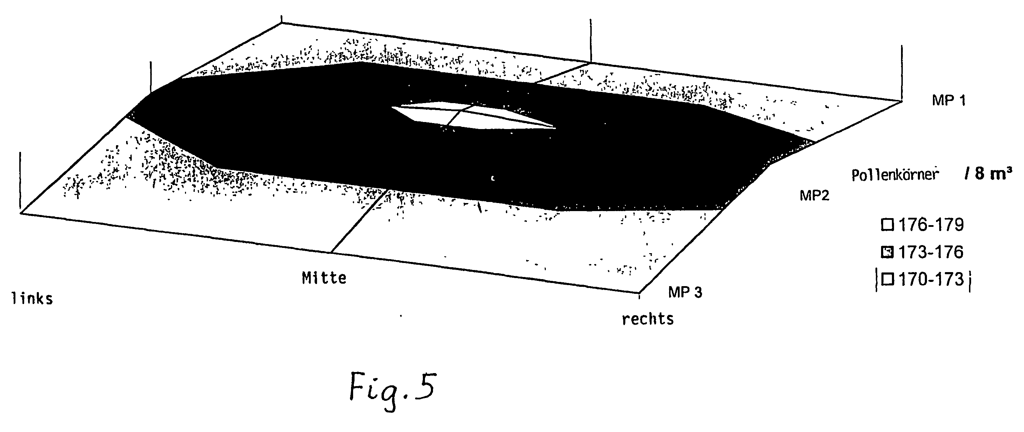

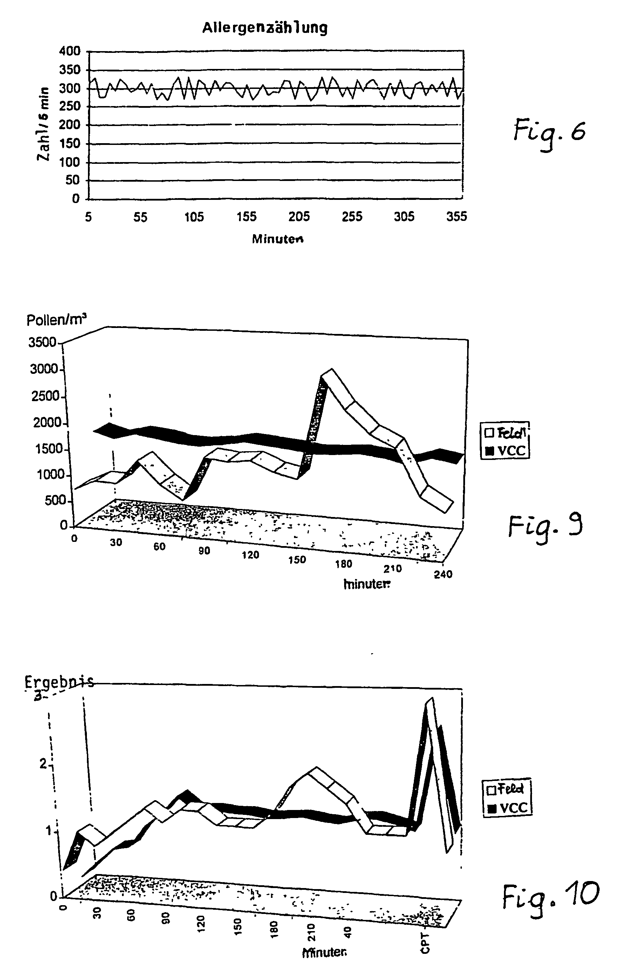

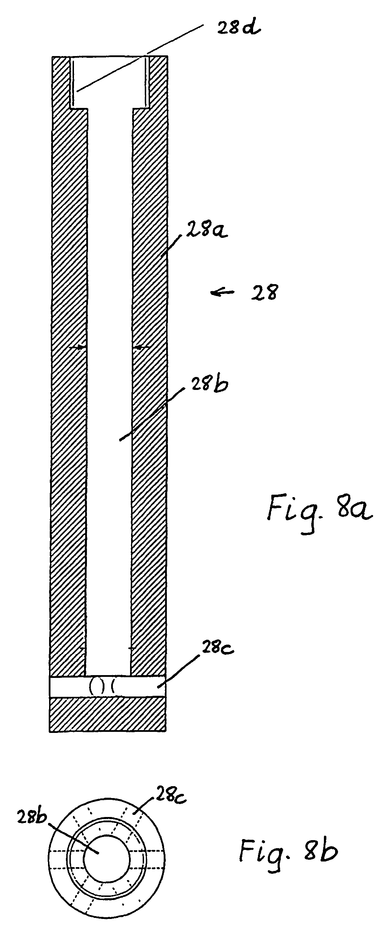

Um Allergen-Testpartikel in die Kammer 20 einzubringen, sind gesonderte Kreisläufe vorgesehen, in denen vorbestimmte Mengen an Allergenpartikeln 40 mittels Druckluft durch Einlässe 25 in die Kammer 20 eingeblasen werden. Für jedes unterschiedliche Allergen (d.h. Graspollen, Birkenpollen, Hauststaubmilben-Faeces etc.) ist ein eigenes Zufuhrsystem installiert, um Kontamination verschiedener Allergene zu vermeiden. Echte Hausstaubmilben-Faeces oder Pollenkörner werden automatisch vom äußeren Arbeitsbereich 30 in die Kammer 20 zugeführt. Zuerst erfolgt eine Ansaugung des Allergens in den Kreislauf durch Vakuum, dann wird das jeweilige Allergen unter Druck an die Kammer abgegeben. Der Ausstoß der Allergen-Testpartikel in die Kammer erfolgt mittels spezieller Partikel-Verteiler 28, (unten unter Bezugnahme auf Fig. 8a und 8b näher erläutert), die an den zwei voneinander beabstandeten Einlässen 25 für Allergen-Partikel, zwischen denen ein Luftverteilerrohr 27 angeordnet ist, angebracht sind (siehe Fig. 4). Diese Partikel-Verteiler 28 und die konstant regulierte, leicht turbulente Luftströmung in der Kammer garantieren eine homogene Dispersion des Allergens in der Luft. Es kann eine langsame und kontinuierliche Sedimentation der Allergenteilchen beibehalten werden. Die Sedimentationsrate beträgt etwa 1 m/min bezogen auf Graspollen-Allergen. Gleichzeitige Messung in drei Höhen und an neun verschiedenen Punkten in jeder Höhe der Kammer (unter Verwendung von modifizierten Burkhard-Pollenfallen) zeigen die hohe Beständigkeit der Allergen-Dispersion (Fig. 5). Die Allergen-Beladung ist innerhalb eines langfristigen Tests über mehrere Stunden innerhalb einer Standardabweichung von nur ≅ 5% konstant (Fig. 6).

Der Partikel-Verteiler 28 gemäß den Figuren 8a und 8b weist einen zylindrischen Körper 28a mit einer Axialbohrung 28b auf, die am oberen Ende erweitert und mit einer Gewindebohrung 28d versehen ist, um sie auf einen Einlaß 25 aufzuschrauben. Am unteren Ende des Körpers 28a erstrecken sich Radialbohrungen 28c von der Axialbohrung 28b nach außen. Allergene werden mittels Druckluft (typisch 3 bar) durch die Axialbohrung und dann durch die Radialbohrungen ins Kammerinnere geblasen, wo sie sich im Luftstrom verteilen.

### Luft-Probennahme

Die Allergen-Beladung der Luft in der Kammer wird während einer Testsitzung auf verschiedene Arten überwacht: Für Routineverfahren wird ein modifizierter Burkhard-Objektträger-Sampler für kontinuierliche volumetrische Aufzeichnungen der Anzahl an Allergen-Teilchen (d.h. Pollenkörner oder Hausstaubmilben-Faeces) pro m³ Luft in 5 min-Intervallen verwendet. Bei einer Strömung von 8 m³/min (der Strömung, die online von einem Thermo-Anemometer FV A645-TH3, der Fa. Alemo, Holzkirchen, DE aufgezeichnet wird), wird der Sampier zugeführt und der mit Kleber beschichtete Objektträger freigelegt. Der Objektträger wird alle 5 min gewechselt und entweder automatisch in einem Lichtmikroskop (z.B. von Olympus, Hamburg, DE) mit Hilfe von Digitalbildanalyse analysiert, oder die Teilchen werden von einem Biologen gezählt. Im Gegensatz zu üblicherweise eingesetzten Probentechniken wird die Strömung der Falle automatisch mit einem Thermo-Anemometer in kurzen Intervallen von 5 s überwacht, und die Berichte werden als Tabellen und Diagramme ausgedruckt. Das gewährleistet eine präzise Auswertung der Objektträger und Berechnung des Allergens in der Luft.

Um den tatsächlichen Ailergengehait der Luft zu überwachen (d.h. ng des zugeführten Haupt-Allergens), wird ein Zyklon-Sampler verwendet, um die Allergen-Teilchen der Luft für spätere Berechnung der Haupt-Allergenkonzentration anzusammeln.

Immunochemische Analyse erfolgt mit einem im Handel erhältlichen ALK Indoor Allergen Analysis Kit® für ELISA-Technik nach den Anweisungen des Herstellers. Schließlich kann mit Laser-Nephelometrie die Anzahl an Teilchen in der Luft kontinuierlich gemessen werden.

### Validierung der Allergen-Beladung

Zur Validierung des Konzeptes muss die VCC mit dem herkömmlichen Test durch Augen-Reizung (OCT) bzw. dem weitverbreiteten Test durch Nasen-Reizung (NCT) und der Umgebungs-Reizsituation (ECS) verglichen werden, wie sie in Feldversuchen oder Park-Versuchen zu finden ist.

Die beim OCT und NCT verwendete Allergen-Quelle ist ein standardisiertes Allergen-Präparat, üblicherweise ein Dialysat von Pollenmaterial oder Hausstaubmilben-Allergen. Für die VCC werden native Pollenkörner oder native Hausstaubmilben-faeces verwendet, die beide von Allergon (Göteborg, Schweden), stammen. Bei ECS sind ebenfalls native Pollenkörner bzw. native Hausstaubmilben-Faecespartikel die Allergen-Quelle. Für die Allergen-Aufbringung bei OCT und NCT wird ein Tropfer oder Vernebler verwendet. Die Einwirkzeit ist sehr kurz (nur wenige Minuten, je nach Tränen-, Nies- und Blinzel-Aktivität. In der VCC und im ECS werden native Teilchen durch die Luft als Aeroplankton abgegeben. Die Einwirkzeit beträgt sowohl bei der VCC als auch im ECS einige Stunden. Es gibt keinen Unterschied im verwendeten Allergen, der Anwendungsweise und der Einwirkdauer zwischen der VCC und dem ECS. Es gibt jedoch einen deutlichen Unterschied zwischen OCT/NCT und ECS bzw. VCC.

Das Ziel der VCC-Technik besteht darin, der Feldsituation so nahe wie möglich zu kommen. Daher werden Allergen-Konzentrationen in Bezug auf die Feldsituation gewählt:

### Untersuchungen mit Pollen-Allergenen:

Untersuchungen mit Patienten, die an saisonaler allergischer Konjunktivitis und/oder saisonaler allergischer Rhinitis leiden, erfordern eine Allergen-Reizung mit Pollen. Für die VCC werden ein Graspollen-Modell und ein Birkenpollen-Modell gewählt (wobei Dactylis Glomerata-Pollenkörner oder Betula Alba-Pollenkörner verwendet werden), da es sich dabei um die häufigsten Allergen-Quellen in Österreich handelt. Bei Feld-Reiz-Untersuchungen sind die Patienten einer großen Vielzahl verschiedener Pollenkonzentrationen im Freien ausgesetzt. Beim Korrelieren der Pollenkonzentration in der Luft und der Symptome des Patienten muss für einen durchschnittlich sensibilisierten Patienten ein Schwellenwert von 250 Pollenkörnern pro m³ Luft angenommen werden. Es wurde auch eine Dosis-abhängige Intensität der Symptome im Bereich zwischen 500 und 2.500 Graspollenkömern pro m³ nachgewiesen. Wenn die verschiedenen Daten der Pollenkonzentration verglichen werden, muss berücksichtigt werden, dass die "Pollen-Warndienste" ihre Daten in einer Höhe von 15 m ermitteln und sich nur auf den Tagesdurchschnitt beziehen. Die Konzentrationen in Bodennähe (1,5 m) sind jedoch viel höher. Das gilt auch für die Spitzenwerte, die viel höher als der Tagesdurchschnitt sind, der auch die fehlende Reizung während der Nacht mitumfaßt. Man muss den Tagesdurchschnitt mit 11 bis 26 multiplizieren, um die Differenz zwischen den Daten aus einer Höhe von 1,5 m und den in 15 m erhaltenen auszugleichen. So entspricht die Konzentration von 2.000 Graspollen pro m³ dem Durchschnitt von 75 bis 180 Pollen pro m³, gemessen mit einer Pollenfalle in einer Höhe von 15 m.

Bei Feld-Reizungs-Untersuchungen sind Patienten einigen hundert bis mehreren tausend Pollenkörnern pro m³ Luft ausgesetzt. Eine Konzentration von etwa 2.000 Pollenkörnern pro m³ Luft ist während der Pollenperiode für mehrere Stunden am Tag sehr wahrscheinlich. Daher ist die bei der VCC verwendete Konzentration üblicherweise 2.000 Pollenkörpern pro m³ Luft, stabil für den gesamten Reizungs-Zeitraum und in jeder Sitzung reproduzierbar. Höhere Konzentrationen erhöhen das Risiko von Bronchial-Anfällen bei allergischen Patienten. Wenn das Untersuchungsprotokoll eine mäßigere Pollen-Reizung erfordert, kann in der VCC eine Konzentration von 1.500 Körnern oder noch weniger verwendet werden.

### Nachweis der Konsistenz bei OCT - ECS - VCC

Um die Konsistenz der klinischen Ergebnisse in verschiedenen Reizungs-Modes zu untersuchen, wurden Testpatienten für 4 h in der VCC mit einer Graspollen-Belastung von 2.000 Körnern/m³ getestet. Am Ende der Testsitzung wurde ein OCT durchgeführt, wobei eine Graspollen-Allergenlösung verwendet wurde (500 BU, ALK - Abello, Dänemark). Zwei Monate später wurde die Untersuchung mit denselben Patienten wiederholt, wobei jedoch anstelle der VCC das Park-Modell als ECS verwendet wurde. Wieder wurde ein abschließender OCT durchgeführt, um die Konsistenz nachzuweisen.

Die Schwankung der Pollenzahl in der VCC und ECS wird in Fig. 9 gezeigt. Die Konzentrationen stimmen in beiden Untersuchungen überein, jedoch ist die Schwankung in der ECS viel ausgeprägter. Subjektive Symptome, die alle 15 min aufgezeichnet werden, sind in der ECS und der VCC sowie in beiden End-OCT vergleichbar (Fig. 10; subjektives Augen-Jucken). Die Gefäßreaktion der Bindehaut wurde unter Verwendung von Bindehaut-Digitalabbildung überwacht. Bilder wurden vor der Testsitzung, nach 4 Stunden ECS bzw. VCC und nach dem OCT gemacht. Es bestand hohe Übereinstimmung zwischen der ECS und der VCC, auch sogar zwischen dem OCT, der ECS und der VCC.

Um die Konsistenz der Ergebnisse nachzuweisen, kann am Ende einer Testsitzung in der VCC eine OCT/NCT durchgeführt werden. Neben der Bestätigung der VCC-Sitzung hat diese Vorgangsweise einen weiteren Vorteil: Es ist möglich, die Reizungs-Stärke mit dem OCT/NCT ohne Risiko am Ende einer langfristigen Reizung zu erhöhen, um zu verifizieren, ob das untersuchte Arzneimittel noch aktiv ist. Eine Erhöhung der Pollenkonzentration auf mehr als 2.000 Körner pro m³ in der VCC würde zu einem höheren Risiko von Bronchialanfällen führen.

Es wurden weitere Untersuchungen an Patienten durch OCT/NCT sowie VCC und ECS durchgeführt. Alle Ergebnisse stimmten überein, was belegt, dass die Ergebnisse von Reizungs-Versuchen in der VCC im Allgemeinen mit Feldbeobachtungen der gleichen Anzahl von Patienten bei einem natürlichen und saisonalen Einwirken vergleichbar sind. Wie erwartet, erlaubt die VCC aufgrund der Menge an Berechnungswerkzeugen präzisere und subtilere Ergebnisse als Untersuchungen im Feld.

### Untersuchungen mit Hausstaubmilben-Allergenen:

Untersuchungen mit Patienten, die an ganzjähriger allergischer Konjunktivitis und/oder ganzjähriger allergischer Rhinitis leiden, erfordern in Hinblick darauf, dass die spezifische Sensibilisierung für Hausstaubmilben-Allergen zu belegen ist, einen Allergen-Test mit Hausstaubmilben. Für die VCC wird ein Modell gewählt, bei dem die Faeces von Dermatophagoides Pteronyssinus verwendet werden, da dies die häufigste Quelle von Hausstaubmilben-Allergie in Österreich ist.

Üblicherweise wird der Allergen-Gehalt eines Raums durch Teppich- und Bodenstaub-Proben definiert, jedoch gelangen Hausstaubmilben-Allergene auch in die Luft, und die Konzentration der Allergene in der Luft ist von grundlegendem Interesse für den sensibilisierten Patienten. Berichte über die Hausstaubmilben-Allergenkonzentration in der Luft sind in der Literatur rar. Sakaguchi et al. "Measurement of allergens associated with dust mite allergy", Int. Arch. Allergy Appl. Immunol. 1989, 90, Seiten 190-193 haben in verschiedenen Haushalten Konzentrationen zwischen 0,03 und 30 ng pro m³ festgestellt. Der Anmelder konnte feststellen, dass der Schwellenwert für den durchschnittlichen sensibilisierten Patienten 10 ng beträgt; von einer Konzentration von 130 ng aufwärts muss man mit einer erhöhten Neigung zu Bronchialanfällen rechnen.

Bei natürlichem Einwirken ist der mittlere Durchmesser von Teilchen, die Haussraubmitben-Attergen enthalten, etwa 20 µm groß. Diese Teilchen sind jedoch nicht stabil, sie zerfallen in mehrere kleinere Teilchen mit einer Größe von 5 µm oder noch weniger. Daher ist es nicht möglich, die Allergen-Beladung zu berechnen, indem nur die Teilchen pro m³ Luft gezählt werden (wie es bei Pollen-Allergenen möglich ist), ohne dass gleichzeitig immunochemische Analyse durchgeführt wird, wie zuvor beschrieben.

### Erhalt klinischer Ergebnisse

Während der Reizung in der Kammer werden die Patienten wiederholt in kurzen Intervallen (üblicherweise 15 oder 30 min) untersucht. Um optimale Daten zu erhalten, werden nicht nur subjektive Symptome registriert, sondern auch objektive Daten gewonnen. Die subjektiven Symptome werden online von den Patienten selbst in eine Datenbank eingegeben. Tests der Lungenfunktion, Nasenfunktion, Produktion von Nasensekret usw. werden von einem Assistenten durchgeführt und bewertet, der die Ergebnisse ebenfalls in die Datenbank eingibt. Jede einzelne Untersuchung muss innerhalb einer Minute durchgeführt werden, um einen vollständigen Untersuchungsdurchgang eines jeden Patienten innerhalb von 15 min zu gewährleisten.

### Schlussfolgerung

Beim Vergleich von Ergebnissen der herkömmlichen Reizungs-Tests, Feld-Untersuchungen und der VCC werden übereinstimmende Ergebnisse festgestellt, aber die Reproduzierbarkeit fällt fraglos zugunsten der VCC aus. Umfassende klinische Untersuchungen konnten die Qualität der VCC beim Testen zahlreicher antiallergener Verbindungen bewiesen.

## Patentansprüche

1. Allergie-Testkammer, mit zumindest einem Einlaß zur Zufuhr von Luft in das Kammerinnere und zumindest einem Auslaß (24) zur Absaugung von mit Allergen-Testpartikeln beladener Luft aus dem Kammerinneren, wobei der Einlaß zur Zufuhr von Luft vom Auslaß (24) beabstandet ist, **dadurch gekennzeichnet, daß** der zumindest eine Einlaß zur Zufuhr von Luft durch zumindest einen Einlaß (23) zur Zufuhr von allergenfreier Luft, insbesondere Frischluft, in das Kammerinnere gebildet ist, welcher mit einer Luftstrom-Verteilungseinrichtung (26,27) versehen ist, daß die Allergie-Testkammer (20) weiters zumindest einen Einlaß (25) für Allergen-Testpartikel, wie z.B. Pflanzenpollen oder Milbenkot, in das Kammerinnere aufweist, und daß der Auslaß (24) zur Absaugung von mit Allergen-Testpartikeln beladener Luft vom Einlaß (25) für Allergen-Testpartikel beabstandet ist.

2. Allergie-Testkammer nach Anspruch 1, **dadurch gekennzeichnet, daß** der zumindest eine Einlaß (23) für allergenfreie Luft ein Luftzufuhrrohr (27) mit einer Vielzahl von Durchgangsöffnungen in der Rohrwand umfaßt.

3. Allergie-Testkammer nach Anspruch 1 oder 2, **dadurch gekennzeichnet, daß** die Luftstrom-Verteilungseinrichtung in verschiedene Richtungen gerichtete Luftdüsen, vorzugsweise Kugeldüsen (26), umfaßt.

4. Allergie-Testkammer nach Anspruch 1 oder 2, **dadurch gekennzeichnet, daß** die Luftstrom-Verteilungseinrichtung Ablenkbleche oder Luftverteiler-Kämme umfaßt.

5. Allergie-Testkammer nach einem der vorhergehenden Ansprüche, **dadurch gekennzeichnet, daß** die Einlässe (25; 23) zur Zufuhr von Allergen-Testpartikeln und von allergenfreier Luft voneinander beabstandet sind.

6. Allergie-Testkammer nach einem der Ansprüche 1 bis 4, **dadurch gekennzeichnet, daß** die Einlässe zur Zufuhr von Allergen-Testpartikeln und von allergenfreier Luft ineinander integriert sind.

7. Allergie-Testkammer nach einem der vorhergehenden Ansprüche, **dadurch gekennzeichnet, daß** die Einlässe (25; 23) zur Zufuhr von Allergen-Testpartikeln und von allergenfreier Luft in der Decke der Kammer und/oder in zumindest einer Seitenwand der Kammer in Deckennähe angeordnet sind, und daß der zumindest eine Auslaß (24) zur Absaugung von mit Allergen-Testpartikeln beladener Luft im Boden der Kammer und/oder in zumindest einer Seitenwand der Kammer in Bodennähe angeordnet ist.

8. Allergie-Testkammer nach einem der vorhergehenden Ansprüche, **dadurch gekennzeichnet, daß** in den Strömungsweg der allergenfreien Luft in die Kammer zumindest ein Mehrschicht-Filtersystem zur Reduzierung von Schadstoffen zwischengeschaltet ist.

9. Allergie-Testkammer nach einem der vorgesehenen Ansprüchen, **dadurch gekennzeichnet, daß** eine Vorrichtung zur Deaglomerierung der in die Kammer einzubringenden Allergenpartikel vorgesehen ist.

10. Allergie-Testkammer nach einem der vorhergehenden Ansprüche, **dadurch gekennzeichnet, daß** eine Vorrichtung zur automatischen Dosierung von Allergen-Testpartikeln und ihrer kontinuierlichen oder intermittierenden Beförderung in die Kammer vorgesehen ist, wobei zur Beförderung der Partikel vorzugsweise Druck- oder Unterdrucksysteme vorgesehen sind.

11. Allergie-Testkammer nach Anspruch 10, **dadurch gekennzeichnet, daß** die Vorrichtung zur automatischen Dosierung zumindest eines aus Mikro-Schneckenförderer, Mikro-Zahnradförderer, Mikro-Archimedes-Schraubenförderer, Mikro-Rotations-Siebscheiben, Zentrifugalselektion, elektromagnetischem oder elektrostatischem Dosierfeld umfaßt, oder halbautomatisch mittels Mikro-Dosierlöffel erfolgt.

12. Allergie-Testkammer nach einem der vorgesehenen Ansprüchen, **dadurch gekennzeichnet, daß** an den zumindest einen Einlaß (25) für Allergen-Testpartikel ein Partikel-Verteiler (28) anschließbar ist, wobei der Partikel-Verteiler vorzugsweise so anbringbar ist, daß die Allergen-Testpartikel an den Punkten mit Strömungsinterferenz Null der allergenbeladenen Luft eingebracht werden.

13. Allergie-Testkammer nach Anspruch 12, **dadurch gekennzeichnet, daß** der Partikel-Verteiler (28) einen Körper (28a) mit einer Axialbohrung (28b) und sich von der Axialbohrung zur Außenperipherie des Verteilers erstreckende Radialbohrungen (28c) umfaßt.

14. Allergie-Testkammer nach Anspruch 12, **dadurch gekennzeichnet, daß** der Partikelverteiler einen Körper mit gerichteten kammartigen Austrittsöffnungen aufweist.

15. Allergie-Testkammer nach einem der Ansprüche 12 bis 14 aufweist, **dadurch gekennzeichnet, daß** der Partikel-Verteiler eine Prallplatte nahe dem Einlaß für Allergen-Testpartikel aufweist, wobei die Allergen-Testpartikel mittels Druckluft durch den Einlaß befördert werden.

16. Allergie-Testkammer nach einem der vorgesehenen Ansprüchen, **dadurch gekennzeichnet, daß** ein Klimamodul zur Steuerung von Klimaparametern wie Temperatur, Feuchtigkeit, Luftumsatz etc. der in das Kammerinnere zugeführten allergenfreien Luft vorgesehen ist, wobei das Klimamodul vorzugsweise über einen Echtzeit-Regelkreis zur Steuerung der Parameter verfügt.

17. Allergie-Testkammer nach einem der vorhergehenden Ansprüche, **dadurch gekennzeichnet, daß** der barometrische Druck in der Kammer gegenüber dem barometrischen Druck außerhalb der Kammer reduziert ist.

18. Allergie-Testkammer nach einem der vorhergehenden Anspruche, **dadurch gekennzeichnet, daß** zumindest ein Meßgerät zur Identifizierung repräsentativer Parameter der eingebrachten Allergen-Testpartikeln vorgesehen ist.

19. Allergie-Testkammer nach Anspruch 18, **dadurch gekennzeichnet, daß** zumindest ein Meßgerät zur Messung der Partikel-Konzentration der Luft im Kammerinneren vorgesehen ist.

20. Allergie-Testkammer nach Anspruch 18, **dadurch gekennzeichnet, daß** für jede Versuchsperson in der Kammer zumindest ein Meßgerät zur Messung der individuellen Allergen-Belastung vorgesehen ist.

21. Allergie-Testkammer nach Anspruch 18, **dadurch gekennzeichnet, daß** zumindest ein Partikel-Sammelgerät zur Kumulierung der über eine Zeiteinheit eingebrachten absoluten Partikel-Menge zwecks anschließender Analyse essentieller Allergenparameter vorgesehen ist.

## Claims

1. An allergy test chamber having at least one inlet for supplying air to the interior of said chamber and at least one outlet (24) for sucking air loaded with allergen test particles out of the interior of said chamber, said inlet for supplying air being spaced apart from said outlet (24), **characterised in that** said at least one inlet for supplying air is formed by at least one inlet (23) for supplying allergen-free air, in particular fresh air, to the interior of said chamber, which inlet is equipped with an air-flow distributing device (26, 27), **in that** said allergy test chamber (20) further comprises at least one inlet (25) for supplying allergen test particles, such as plant pollen or mite faeces, to the interior of said chamber, and **in that** said outlet (24) for sucking off air loaded with allergen test particles is spaced apart from said inlet (25) for allergen test particles.

2. An allergy test chamber according to claim 1, **characterised in that** said at least one inlet (23) for allergen-free air comprises an air supply pipe (27) having a plurality of passage openings formed in the pipe wall.

3. An allergy test chamber according to claim 1 or 2, **characterised in that** said air-flow distributing device comprises air nozzles, preferably ball nozzles (26), oriented in different directions.

4. An allergy test chamber according to claim 1 or 2, **characterised in that** said air-flow distributing device comprises baffle plates or air distribution combs.

5. An allergy test chamber according to any one of the preceding claims, **characterised in that** said inlets (25; 23) for supplying allergen test particles and allergen-free air are spaced apart from each other.

6. An allergy test chamber according to any one of claims 1 to 4, **characterised in that** said inlets for supplying allergen test particles and allergen-free air are intergrated one within the other.

7. Allergy test chamber according to any of the previous claims, **characterised in that** said inlets (25; 23) for supplying allergen test particles and allergen-free air are arranged in the ceiling of said chamber and/or in at least one sidewall of said chamber close to the ceiling, and **in that** said at least one outlet (24) for sucking off air loaded with allergen test particles is provided in the bottom of said chamber and/or in at least one sidewall of said chamber close to the bottom.

8. Allergy test chamber according to any one of the preceding claims, **characterised in that** at least one multi-layer filter system for the reduction of pollutants is inserted in the flow path of allergen-free air into said chamber.

9. Allergy test chamber according to any one of the preceding claims, **characterised in that** a device for de-agglomerating the allergen particles to be introduced into said chamber is provided.

10. Allergy test chamber according to any one of the preceding claims, **characterised in that** a device for the automatic dosage of allergen test particles and their continuous or intermittent transportation into said chamber is provided, with pressure or partial-vacuum systems preferably being provided for transporting the particles.

11. Allergy test chamber according to claim 10, **characterised in that** said device for the automatic dosage comprises at least one of screw micro-conveyors, gear micro-conveyors, Archimedean-screw micro-conveyors, rotary micro-sieve disks, centrifugal selection, electromagnetic or electrostatic dosing field, or is earned out semi-automatically using a micro dosing-spoon.

12. Allergy test chamber according to any one of the preceding claims, **characterised in that** a particle distributor (28) is connectable to said at least one inlet (25) for allergen test particles, said particle distributor preferably being mountable in such a way that the allergen test particles are introduced at the points with zero flow interference of the allergen-laden air.

13. Allergy test chamber according to claim 12, **characterised in that** said particle distributor (28) comprises a body (28a) having an axial bore (28b) and radial bores (28c) extending from said axial bore to the outer periphery of said distributor.

14. Allergy test chamber according to claim 12, **characterised in that** said particle distributor comprises a body having aligned, comb-like exit openings.

15. Allergy test chamber according to any one of claims 12 to 14, **characterised in that** said particle distributor comprises a baffle plate close to said inlet for allergen test particles, the allergen test particles being conveyed through said inlet by means of compressed air.

16. Allergy test chamber according to any one of the preceding claims, **characterised in that** a climate module is provided for controlling climate parameters like temperature, humidity, throughput of air, etc. of the allergen-free air supplied into said chamber, said climate module preferably being equipped with a real-time control circuit for controlling the parameters.

17. Allergy test chamber according to any one of the preceding claims, **characterised in that** the barometric pressure inside said chamber is reduced as compared to the barometric pressure outside said chamber.

18. Allergy test chamber according to any one of the preceding claims, **characterised in that** at least one measuring instrument is provided for identifying representative parameters of the allergen test particles introduced.

19. Allergy test chamber according to claim 18, **characterised in that** at least one measuring instrument for measuring the particle concentration in the air is provided inside said chamber.

20. Allergy test chamber according to claim 18, **characterised in that** at least one measuring instrument each for every test subject in said chamber is provided for measuring the individual allergen load.

21. Allergy test chamber according to claim 18, **characterised in that** at least one particle collector is provided for accumulating the absolute particle quantity introduced in the course of one time unit for the purpose of subsequently analysing essential allergen parameters.

## Revendications

1. Chambre de test d'allérgie avec au moins une entrée pour l'alimentation en air de l'intérieur de la chambre et au moins une sorte (24) pour l'aspiration d'air chargé de particules allergènes de test de l'intérieur de la chambre, l'entrée pour l'alimentation en air étant éloignée de la sortie (24), **caractérisée en ce que** l'au moins une entrée pour l'alimentation en air est formée d'au moins une entrée (23) pour l'alimentation en air dépourvu d'allergènes, en particulier d'air frais, de l'intérieur de la chambre, l'entrée étant munie d'un dispositif de distribution du flux d'air (26, 27), **en ce que** la chambre de test d'allérgie (20) comprend en plus une entrée (25) pour des particules allergènes de test, par exemple des pollens de plantes ou des excréments d'acariens, et **en ce que** la sortie (24) pour l'aspiration d'air chargé de particules allergènes de test est éloignée de l'entrée (25) pour des particules allergènes de test.

2. Chambre de test d'allérgie selon la revendication 1, **caractérisée en ce que** l'au moins une entrée (23) pour l'air dépourvu d'allergènes comprend un tuyau d'alimentation en air (27) avec une pluralité d'orifices de passage formés dans la paroi du tuyau.

3. Chambre de test d'allérgie selon la revendication 1 ou 2, **caractérisée en ce que** le dispositif de distribution du flux d'air comprend des buses d'air, de préférence des buses sphériques (26), orientées dans des directions différentes.

4. Chambre de test d'allérgie selon la revendication 1 ou 2, **caractérisée en ce que** le dispositif de distribution du flux d'air comprend des déflecteurs ou des peignes de distribution d'air.

5. Chambre de test d'allérgie selon l'une quelconque des revendications précédentes, **caracterisée en ce que** les entrées (25; 23) pour l'alimentation en particules allergènes de test et en air dépourvu d'allergènes sont eloignées l'une de l'autre.

6. Chambre de test d'allérgie selon l'une quelconque des revendications 1 à 4, **caractérisée en ce que** les entrées pour l'alimentation en particules allergènes de test et en air dépourvu d'allergènes sont intégrées l'une dans l'autre.

7. Chambre de test d'allérgie selon l'une quelconque des revendications précédentes, **caracterisée en ce que** les entrées (25, 23) pour l'alimentation en particules allergènes de test et en air dépourvu d'allergènes sont disposées dans le plafond de la chambre et/ou dans au moins une paroi latérale de la chambre près du plafond et **en ce que** l'au moins une sortie (24) pour l'aspiration d'air chargé de particules allergènes de test est disposée dans le fond de la chambre et/ou dans au moins une paroi latérale de la chambre près du fond.

8. Chambre de test d'allérgie selon l'une quelconque des revendications précédentes, **caracterisée en ce qu'** un système de filtrage multicouche pour diminuer le taux de polluants est inséré dans le chemin du flux d'air dépourvu d'allergènes entrant dans la chambre.

9. Chambre de test d'allérgie selon l'une quelconque des revendications précédentes, **caracterisée en ce qu'** un dispositif est prévu pour la desagglomération des particules allergènes de test à introduire dans la chambre.

10. Chambre de test d'allérgie selon l'une quelconque des revendications précédentes, **caractérisée en ce qu'** un dispositif est prévu pour le dosage automatique des particules allergènes de test et pour leur transport continu ou intermittent à la chambre, des systèmes à pression ou à dépression étant prévus de préférence pour le transport des particules.

11. Chambre de test d'allérgie selon la revendication 10, caractensée en ce que le dispositif pour le dosage automatique comprend au moins un/une de mini-convoyeur à vis, mini-convoyeur à engrenages, mini-convoyeur à vis d'Archimède, micro-tamis rotatif à disques, sélection centrifuge, champ électromagnétique ou électrostatique de dosage, ou est effectué de façon semi-automatique avec une micro-cuillère de dosage.

12. Chambre de test d'allérgie selon l'une quelconque des revendications précédentes, **caractérisée en ce qu'** un distributeur de particules (28) est connectable à l'au moins une entrée (25) pour des particules allergènes de test, le distributeur de particules étant de préférence montable de sorte que les particules allergènes de test sont introduites aux points où l'interférence de flux de l'air chargé d'allergènes égale zéro.

13. Chambre de test d'allérgie selon la revendication 12, **caracterisée en ce que** le distributeur de particules (28) comprend un corps (28a) avec un trou axial (28b) et des trous radiaux (28c) s'étendant du trou axiale à la périphérie extérieure du distributeur.

14. Chambre de test d'allérgie selon la revendication 12, **caracterisée en ce que** le distributeur de particules possède un corps avec des orifices de décharge alignées et en forme de peigne.

15. Chambre de test d'allérgie selon l'une quelconque des revendications 12 à 14, **caracterisée en ce que** le distributeur de particules possède une plaque de déviation près de l'entrée pour des particules allergènes de test, les particules allergènes de test étant transportées à travers l'entrée à l'aide d'air comprimé.

16. Chambre de test d'allérgie selon l'une quelconque des revendications précédentes, **caracterisée en ce qu'** un module climatique est prévu pour le réglage de paramètres climatiques tels que la température, l'humidité, le débit d'air etc. de l'air dépourvu d'allergènes introduit dans l'intérieur de la chambre, le module climatique de préference étant équipé d'un circuit de régulation fonctionnant en temps réel pour le réglage des paramètres

17. Chambre de test d'allérgie selon l'une quelconque des revendications précédentes, **caracterisée en ce que** la pression barométrique dans la chambre est réduite par rapport à la pression barométrique hors de la chambre

18. Chambre de test d'allérgie selon l'une quelconque des revendications précédentes, **caracterisée en ce qu'** au moins un instrument de mesure est prévu pour l'identification de paramètres représentatifs des particules allergènes de test introduites.

19. Chambre de test d'allérgie selon la revendication 18, **caracterisée en ce qu'** au moins un instrument de mesure est prévu pour mesurer la concentration en particules de l'air dans intérieur de la chambre

20. Chambre de test d'allérgie selon la revendication 18, **caracterisée en ce qu'** au moins un instrument de mesure est prévu par sujet d'étude dans la chambre pour mesurer la charge allergénique individuelle

21. Chambre de test d'allérgie selon la revendication 18, **caractérisée en ce qu'** un collecteur de particules est prévu pour accumuler la quantité absolue de particules introduite pendant une unité de temps afin d'éffectuer une analyse subséquente de paramètres essentiels de l'allergène.
